(19) **Europäisches Patentamt**
**European Patent Office**
**Office européen des brevets**

(11) **EP 3 509 068 A1**

(12) **EUROPEAN PATENT APPLICATION**

(43) Date of publication:
**10.07.2019 Bulletin 2019/28**

(21) Application number: **18182037.4**

(22) Date of filing: **05.07.2018**

(51) Int Cl.:
*G16H 20/30* (2018.01)         *A61B 5/11* (2006.01)
*A61H 3/00* (2006.01)          *A61H 1/02* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.09.2017   KR 20170121360**

(71) Applicant: **Samsung Electronics Co., Ltd.**
**Gyeonggi-do 16677 (KR)**

(72) Inventors:
• **Jang, Jun-Won**
  **16678 Gyeonggi-do (KR)**
• **Kim, Kyung-Rock**
  **16678 Gyeonggi-do (KR)**
• **Shim, Youngbo**
  **16678 Gyeonggi-do (KR)**
• **Lee, Jusuk**
  **16678 Gyeonggi-do (KR)**
• **Lim, Bokman**
  **16678 Gyeonggi-do (KR)**

(74) Representative: **Hylarides, Paul Jacques et al**
**Arnold & Siedsma**
**Bezuidenhoutseweg 57**
**2594 AC Den Haag (NL)**

(54) **METHOD AND APPARATUS FOR UPDATING PERSONALIZED GAIT POLICY**

(57)     A walking assistance device may be controlled using a personalized gait policy. A preset event is detected during a current gait cycle based on state information associated with a motion of the walking assistance device, a reward value of state information associated with the motion is evaluated at a point in time at which the event is detected, and a personalized gait policy is updated based on state information associated with the motion when updating of the personalized gait policy is determined to be required based on the reward value.

**FIG. 1**

140
130
100
110
120

EP 3 509 068 A1

**Description**

BACKGROUND

1. Field

Some example embodiments relate to a method and/or apparatus for updating a personalized gait policy. For example, at least some example embodiments relate to a method and/or apparatus for updating a personalized gait policy while a user keeps walking.

2. Description of the Related Art

With the onset of aging society, a growing number of persons have inconveniences and pain in walking due to weak muscles and malfunctioning joint issues. Accordingly, there is an increasing interest in a walking assistance device for those who are uncomfortable with their muscles and unable to walk easily. Also, walking assistance devices for enhancing a strength of a human body for the military use are being developed.

SUMMARY

Some example embodiments relate to a method of controlling a walking assistance device.
In some example embodiments, the method includes generating an assist profile for controlling the walking assistance device during a current gait cycle based on a personalized gait policy, the personalized gait policy being generated by, receiving state information associated with a motion of the walking assistance device measured in a previous gait cycle, the previous gait cycle being a gait cycle occurring before the current gait cycle, detecting a first event during the current gait cycle, evaluating a reward value of state information associated with the motion at a point in time at which the first event is detected, and selectively updating the personalized gait policy based on the state information associated with the motion and the reward value; and controlling the walking assistance device based on the assist profile.
In some example embodiments, the state information associated with the motion includes a change trajectory of one or more of a hip joint angle, a knee joint angle, and an ankle joint angle of the walking assistance device.
In some example embodiments, the detecting the first event includes: detecting one of: an event that a hip joint angle of a lead leg of the walking assistance device is maximum; an event that both legs of the walking assistance device cross; an event that the lead leg of the walking assistance device is in contact with a ground; and an event that a follow leg of the walking assistance device leaves the ground.
In some example embodiments, the previous gait cycle is from a point in time at which a previous event corresponding to the first event is detected to a point in time at which the first event is detected, and the current gait cycle is from the point in time at which the first event is detected to a point in time at which a next event corresponding to the first event is detected.
In some example embodiments, the reward value relates to state information about the motion based on a previous assist profile generated based on a previous gait policy, the previous gait policy being a gait policy in effect prior to the personalized gait policy.
In some example embodiments, the updating of the personalized gait policy includes: updating a neural network of the personalized gait policy by changing a weight of a parameter that constitutes the neural network of the previous gait policy.
In some example embodiments, the updating of the personalized gait policy includes: updating a left-leg gait policy for a left leg of the walking assistance device; and updating a right-leg gait policy for a right leg of the walking assistance device.
In some example embodiments, the updating of the left-leg gait policy includes at least one of: updating a left-flexion gait policy for a flexion mode of the left leg of the walking assistance device; and updating a left-extension gait policy for an extension mode of the left leg of the walking assistance device.
In some example embodiments, the generating of the assist profile includes: generating the assist profile based on the state information associated with the motion and the personalized gait policy.
In some example embodiments, the controlling of the walking assistance device includes: detecting a second event during the current gait cycle; and controlling, from a point in time at which the second event is detected, the walking assistance device to operate based on the assist profile.
In some example embodiments, the controlling of the walking assistance device includes: instructing at least one driver of the walking assistance device to generate a torque based on the assist profile.
In some example embodiments, the controlling of the walking assistance device includes: providing a functional electrical stimulus to a user based on the assist profile.

In some example embodiments, the receiving of the state information includes: receiving measurement values from at least one sensor of the walking assistance device, the measurement values being the state information associated with the motion, wherein the detecting the first event detects the first event based on the measurement values.

In some example embodiments, the reward value includes one or more of a gait symmetry of state information associated with the motion, a power consumption amount of state information associated with the motion, a gait stability of state information associated with the motion, metabolic cost, and external feedback.

In some example embodiments, the walking assistance device is configured to calculate the gait symmetry based on one or more of a step length and a step duration of a user.

In some example embodiments, the walking assistance device is configured to generate the personalized gait policy.

In some example embodiments, the walking assistance device is configured to communicate with a server, and the updating the personalized gait policy includes: updating, by the server, update the personalized gait policy based on the state information.

In some example embodiments, the method further includes receiving, by the walking assistance device, the personalized gait policy from the server.

In some example embodiments, the controlling of the walking assistance device includes: transmitting, by the server, the assist profile to the walking assistance device.

In some example embodiments, the controlling of the walking assistance device includes: generating, by the server, a control signal for controlling a driver of the walking assistance device based on the assistance profile; and transmitting the control signal to the walking assistance device to control the walking assistance device.

Some example embodiments relate to a non-transitory computer-readable medium storing computer readable instructions, which when executed by a computer, configure the computer to control a walking assistance device.

Some example embodiments relate to a walking assistance device.

In some example embodiments, the walking assistance device includes a memory configured to store a program for controlling the walking assistance device; and a processor configured to execute the program to, generate an assist profile to control the walking assistance device during a current gait cycle based on a personalized gait policy, the personalized gait policy being generated by, receiving state information associated with a motion of the walking assistance device measured in a previous gait cycle, the previous gait cycle being a gait cycle occurring before the current gait cycle, detecting a first event during the current gait cycle, evaluating a reward value of state information associated with the motion at a point in time at which the first event is detected, and selectively updating the personalized gait policy based on the state information associated with the motion and the reward value; and control the walking assistance device based on the assist profile.

In some example embodiments, the processor of the walking assistance device is configured to update the personalized gait policy.

In some example embodiments, the walking assistance device is configured to, receive the personalized gait policy from a server, the server being configured to update the personalized gait policy.

Some example embodiments relate to a method of updating a personalized gait policy to control a walking assistance device.

In some example embodiments, the method includes receiving state information associated with a motion of the walking assistance device measured in a previous gait cycle; detecting an event during a current gait cycle; evaluating a reward value of state information associated with the motion at a point in time at which the event is detected; selectively updating the personalized gait policy based on the state information associated with the motion and the reward value; generating an assist profile or an assist profile parameter based on the personalized gait policy; and transmitting, to the walking assistance device, the assist profile or the assist profile parameter to control the walking assistance device.

Some example embodiments relate to a server configured to update a personalized gait policy to control a walking assistance device during a current gait cycle.

In some example embodiments, the server includes a memory configured to store a program to control the walking assistance device; and a processor configured to execute the program to, receive state information associated with a motion of the walking assistance device measured in a previous gait cycle, the previous gait cycle being a gait cycle occurring before the current gait cycle, detect an event during the current gait cycle, evaluate a reward value of state information associated with the motion at a point in time at which the event is detected; selectively update the personalized gait policy based on the state information associated with the motion and the reward value; generate an assist profile or an assist profile parameter based on the personalized gait policy; and transmit, to the walking assistance device, the assist profile or the assist profile parameter to control the walking assistance device.

Some example embodiments relate to a method of updating a personalized gait policy to control a walking assistance device during a current gait cycle.

In some example embodiments, the method includes receiving state information of the walking assistance device about a previous gait cycle, the previous gait cycle being a gait cycle occurring before the current gait cycle; evaluating the personalized gait policy based on the state information in response to an occurrence of an event; and selectively updating the gait policy during a walking of the walking assistance device based on the evaluation.

In some example embodiments, the evaluating of the personalized gait policy includes: evaluating the personalized gait policy based on one or more of a gait symmetry, a power consumption amount, a gait stability, metabolic energy, and external feedback.

In some example embodiments, the method is performed by a server, the server configured to communicate with the walking assistance device.

Additional aspects of example embodiments will be set forth in part in the description which follows and, in part, will be apparent from the description, or may be learned by practice of the disclosure.

## BRIEF DESCRIPTION OF THE DRAWINGS

These and/or other aspects will become apparent and more readily appreciated from the following description of example embodiments, taken in conjunction with the accompanying drawings of which:

FIGS. 1 and 2 illustrate an example of a walking assistance device according to at least one example embodiment;
FIG. 3 is a diagram illustrating a configuration of a walking assistance device according to at least one example embodiment;
FIG. 4 is a flowchart illustrating a method of controlling a walking assistance device according to at least one example embodiment;
FIG. 5 is a graph showing an example of an assist torque profile according to at least one example embodiment;
FIG. 6 is a diagram illustrating a configuration of a gait policy updating apparatus according to at least one example embodiment;
FIG. 7 is a flowchart illustrating a method of generating a gait policy according to at least one example embodiment;
FIG. 8 is a graph showing an example of state information associated with a motion of a walking assistance device according to at least one example embodiment;
FIG. 9 is a graph showing an example of an event point in time detected based on state information associated with a motion of a walking assistance device according to at least one example embodiment;
FIG. 10 is a flowchart illustrating a method of receiving external feedback according to at least one example embodiment;
FIG. 11 is a flowchart illustrating a method of generating a personalized gait policy according to at least one example embodiment;
FIG. 12 illustrates an example of a personalized gait policy according to at least one example embodiment;
FIG. 13 is a flowchart illustrating a method of controlling a walking assistance device based on an assist profile according to at least one example embodiment;
FIG. 14 is a graph showing an example of an assist torque profile according to at least one example embodiment;
FIG. 15 is a graph showing an example of a trajectory of an assist profile according to at least one example embodiment;
FIG. 16 illustrates a relationship between a walking assistance device and a gait policy updating apparatus according to at least one example embodiment;
FIG. 17 illustrates a gait policy updating apparatus configured as a server according to at least one example embodiment;
FIG. 18 is a diagram illustrating a gait policy updating apparatus included in a walking assistance device according to at least one example embodiment;
FIG. 19 is a flowchart illustrating a method of controlling a walking assistance device based on a target mode according to at least one example embodiment;
FIG. 20 is a flowchart illustrating a method of outputting a notification of requesting updating of a personalized gait policy; and
FIGS. 21 through 23 illustrate examples of a walking assistance device according to at least one example embodiment.

## DETAILED DESCRIPTION

Hereinafter, some example embodiments will be described in detail with reference to the accompanying drawings. Regarding the reference numerals assigned to the elements in the drawings, it should be noted that the same elements will be designated by the same reference numerals, wherever possible, even though they are shown in different drawings. Also, in the description of example embodiments, detailed description of well-known related structures or functions will be omitted when it is deemed that such description will cause ambiguous interpretation of the present disclosure.

It should be understood, however, that there is no intent to limit example embodiments to the particular example embodiments disclosed herein. On the contrary, the example embodiments are to cover all modifications, equivalents, and alternatives falling within the scope of the example embodiments. Like numbers refer to like elements throughout the description of the figures.

In addition, terms such as first, second, A, B, (a), (b), and the like may be used herein to describe components. Each of these terminologies is not used to define an essence, order or sequence of a corresponding component but used merely to distinguish the corresponding component from other component(s). It should be noted that if it is described in the specification that one component is "connected", "coupled", or "joined" to another component, a third component may be "connected", "coupled", and "joined" between the first and second components, although the first component may be directly connected, coupled or joined to the second component.

The terminology used herein is for the purpose of describing particular example embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

As used herein, the term "and/or" includes any and all combinations of one or more of the associated listed items. Expressions such as "at least one of" and "one of" when preceding a list of elements, modify the entire list of elements and do not modify the individual elements of the list. Thus, for example, both "at least one of A, B, or C" and "A, B, and/or C" means either A, B, C or any combination thereof.

Various example embodiments will now be described more fully with reference to the accompanying drawings in which some example embodiments are shown. In the drawings, the thicknesses of layers and regions are exaggerated for clarity.

Hereinafter, a general concept of a walking assistance device according to at least one example embodiment is described.

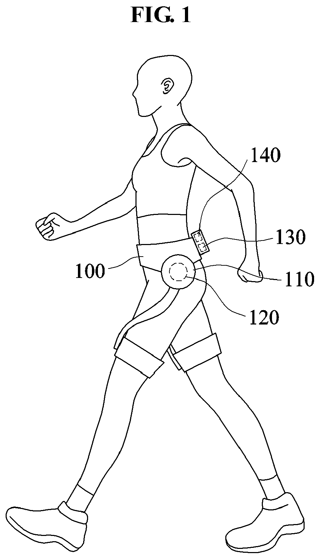

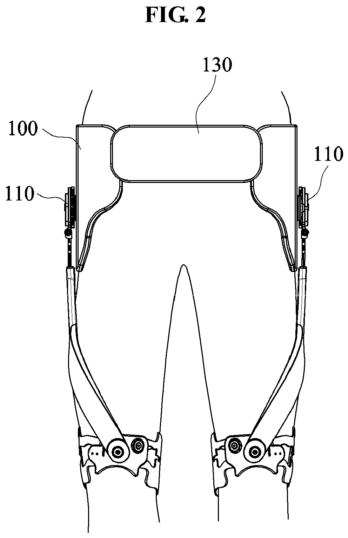

FIGS. 1 and 2 illustrate an example of a walking assistance device according to at least one example embodiment.

Referring to FIG. 1, a walking assistance device 100 is provided to a user to assist walking of the user. The walking assistance device 100 may be a wearable device.

Although FIG. 1 illustrates a hip-type walking assistance device, it is provided as an example only. The walking assistance device 100 may be provided in a type of supporting the entire lower body or a type of supporting a portion of the lower body. Also, the walking assistance device 100 may be provided in one of a type of supporting a portion of the lower body, a type of supporting up to a knee, a type of supporting up to an ankle, and a type of supporting the entire body.

Example embodiments described herein may be applied to a hip type and also be applied to any type of devices that assist walking of the user.

The walking assistance device 100 includes a driver 110, a sensor 120, an inertial measurement unit (IMU) 130, and a controller 140.

The driver 110 provides a driving force to one or more hip joints of the user. For example, the driver 110 may be provided on a right hip portion and/or a left hip portion of the user.

The driver 110 may include a motor capable of generating a rotational torque.

The sensor 120 may measure an angle of the hip joint of the user during walking. Information associated with the angle of the hip joint sensed by the sensor 120 may include an angle of a right hip joint, an angle of a left hip joint, a difference between hip joint angles, and a hip joint motion direction. For example, the sensor 120 may be included in the driver 110.

According to an example embodiment, the sensor 120 may include a potentiometer. The potentiometer may sense a right (R) axis joint angle, a left (L) axis joint angle, an R axis joint acceleration, and an L axis joint acceleration based on a walking motion of the user.

The IMU 130 may measure acceleration information and posture information during walking. For example, the IMU 130 may sense each of X axis, Y axis, and Z axis acceleration and X axis, Y axis, and Z axis angular velocities according to a walking motion of the user.

The walking assistance device 100 may detect a point at which a foot of the user lands based on acceleration information measured by the IMU 130.

The walking assistance device 100 may include other sensors, for example, an electromyogram (EMG) sensor and an electroencephalogram (EEG) sensor, capable of sensing a change in a momentum or a biosignal of the user according to the walking motion, in addition to the sensor 120 and the IMU 130.

The controller 140 controls the driver 110 to output an assistance force for assisting walking of the user. For example, the hip-type walking assistance device 100 may include two drivers 110 that are provided to a left hip and a right hip, respectively, and the controller 140 may output control signals for controlling the two drivers 110 to generate a torque.

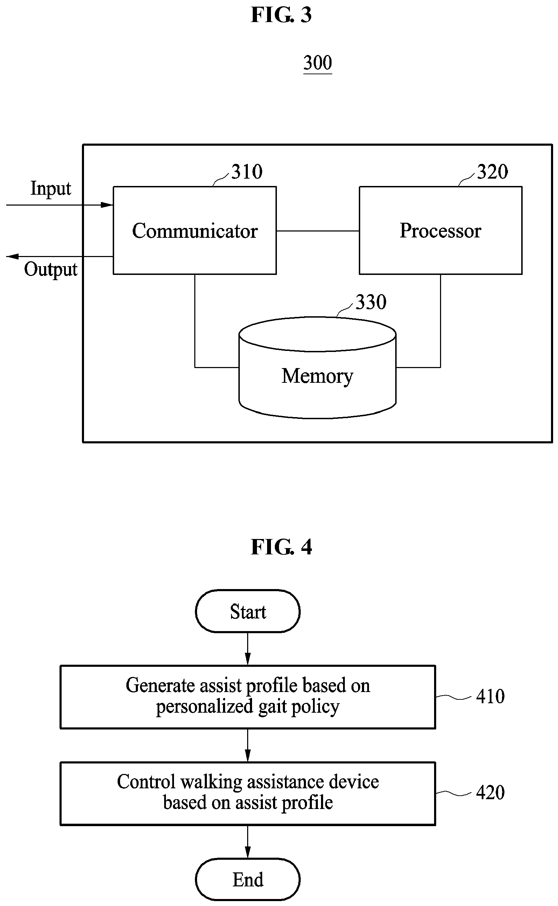

The controller 140 may include a communicator, a processor, and a memory. The controller 140 is further described with reference to FIG. 3.

The driver 110 generates the torque in response to the control signal output from the controller 140. According to an example embodiment, the walking assistance device 100 may include the driver 110 for a right leg and the driver 110 for a left leg. For example, the controller 140 may be designed to control one or more drivers 110. If the controller 140 is configured to control only a single driver 110, a plurality of controllers 140 may be used. As another example, the controller 140 may be designed to control all of the drivers 110.

Although not illustrated, the walking assistance device 100 may include a muscle stimulation device. The muscle stimulation device may generate an electrical signal that stimulates muscles. The controller 140 may control the muscle stimulation device. If muscles of the user are stimulated, the muscles may contract regardless of the user's will and muscular strength may be generated.

## <Walking assistance device that operates based on a gait policy and a method of operating the walking assistance device>

Hereinafter, a walking assistance device that operates based on a gait policy and a method of operating the walking assistance device according to at least one example embodiment will be described with reference to FIGS. 3 and 4.

FIG. 3 is a diagram illustrating a configuration of a controller of a walking assistance device according to at least one example embodiment.

Referring to FIG. 3, a controller 300 includes a communicator 310, a processor 320, and memory 330. The controller 300 may correspond to the controller 140 described above with FIGS. 1 and 2.

The communicator 310 is connected to the processor 320 and the memory 330 to transmit and/or receive data. The communicator 310 may be connected to an external device to transmit and receive data. Hereinafter, transmitting and receiving "A" may represent transmitting and receiving "information or data that indicates A".

The communicator 310 may be configured as a circuitry within the controller 300. For example, the communicator 310 may include an internal bus and an external bus. As another example, the communicator 310 may refer to a component that connects the controller 300 and an external device. The communicator 310 may be an interface. The communicator 310 may receive data from the external device and may transmit the data to the processor 320 and the memory 330. Further, the communicator 310 may include one or more transmitters and/or receivers that include hardware and any necessary software for transmitting or received signals via the associated interface to other network elements in the network environment.

The processor 320 processes data received by the communicator 310 and data stored in the memory 330. The processor 320 may be a data processing device configured as hardware having a circuit with a physical structure for executing desired operations. For example, the desired operations may include instructions or a code included in a program. For example, the data processing device configured as hardware may include a microprocessor, a central processing unit (CPU), a processor core, a multi-core processor, a multiprocessor, an application-specific integrated circuit (ASIC), and a field programmable gate array (FPGA).

The processor 320 may execute a computer-readable code, for example, software, stored in the memory 330, and instructions caused by the processor 320. The memory 330 may contain computer readable code that, when executed by the processor 320, configures the processor 320 as a special purpose processor to generate an assist profile for controlling the walking assistance device 100 during a current gait cycle based on a personalized gait policy, and to control the walking assistance device based on the assist profile.

In some example embodiments, the computer readable code stored in the memory 330, when executed by the processor 320 may further configure the processor 320 to generate the personalized gait policy or receive the personalized gait policy from an external device, such as a server. The personalized gait policy may be generated and/or updated by, receiving state information associated with a motion of the walking assistance device measured in a previous gait cycle, the previous gait cycle being a gait cycle occurring before the current gait cycle, detecting a first event during the current gait cycle, evaluating a reward value of state information associated with the motion at a point in time at which the first event is detected, and selectively updating the personalized gait policy based on the state information associated with the motion and the reward value.

The memory 330 stores data received by the communicator 310 and data processed by the processor 320.

For example, the memory 330 may store a program. The stored program may be a set of syntaxes that are coded to be capable of controlling the walking assistance device 100 and to be executable by the processor 320.

According to an example embodiment, the memory 330 may include at least one volatile memory, non-volatile memory, random access memory (RAM), a flash memory, a hard disk drive, and an optical disk drive.

The memory 330 stores an instruction set, for example, software, for operating the controller 300. The instruction set for operating the controller 300 is executed by the processor 320.

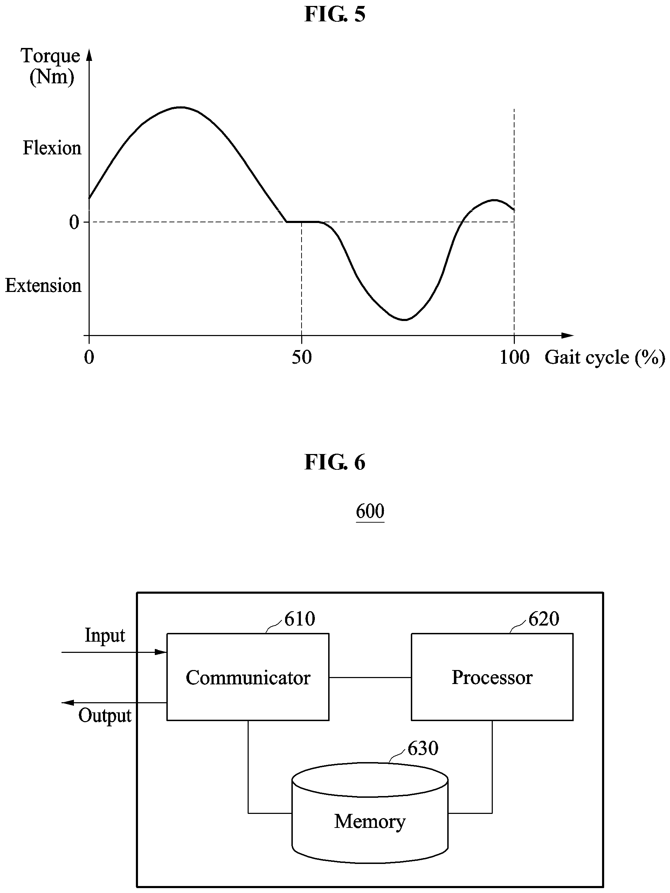

A further description related to the communicator 310, the processor 320, and the memory 330 will be made with reference to FIGS. 4 and 5.

FIG. 4 is a flowchart illustrating a method of controlling a walking assistance device according to at least one example embodiment.

Operations 410 and 420 of FIG. 4 may be performed by the controller 300 of FIG. 3.

In operation 410, the processor 320 generates an assist profile for controlling the walking assistance device 100 based on a personalized gait policy. The processor 320 may generate the assist profile based on state information associated with a motion of the walking assistance device 100 and the personalized gait policy.

The assist profile may be used to control the driver 110 of the walking assistance device 100. For example, the assist profile may include an assist torque profile indicating a torque that is output over time, a joint trajectory profile indicating an angle of a joint that varies over time, and/or a functional electrical stimulation (FES) profile indicating electrical stimulation that is provided to muscles over time. The assist torque profile is further described below with reference to FIG. 5.

In operation 420, the processor 320 controls the walking assistance device 100 based on the assist profile. For example, the processor 320 generates a control signal for controlling the driver 110 using the assist torque profile. The driver 110 outputs a torque corresponding to the control signal. The output torque may assist walking of the user. As another example, the processor 320 may generate a signal for controlling a joint angle using the joint trajectory profile. As another example, the processor 320 may generate a signal for simulating muscles of the user using the FES profile.

The walking assistance device 100 may generate the assist profile based on the personalized gait policy. For example, the personalized gait policy may be generated by updating a default gait policy. The updated personalized gait policy may be stored in the walking assistance device 100. The walking assistance device 100 may generate the assist profile using the personalized gait policy and may use the walking of the user using the assist profile.

The personalized gait policy may be generated in advance by a gait policy updating apparatus. A method of updating, by the gait policy updating apparatus, the personalized gait policy will be described below with reference to FIGS. 6 through 12.

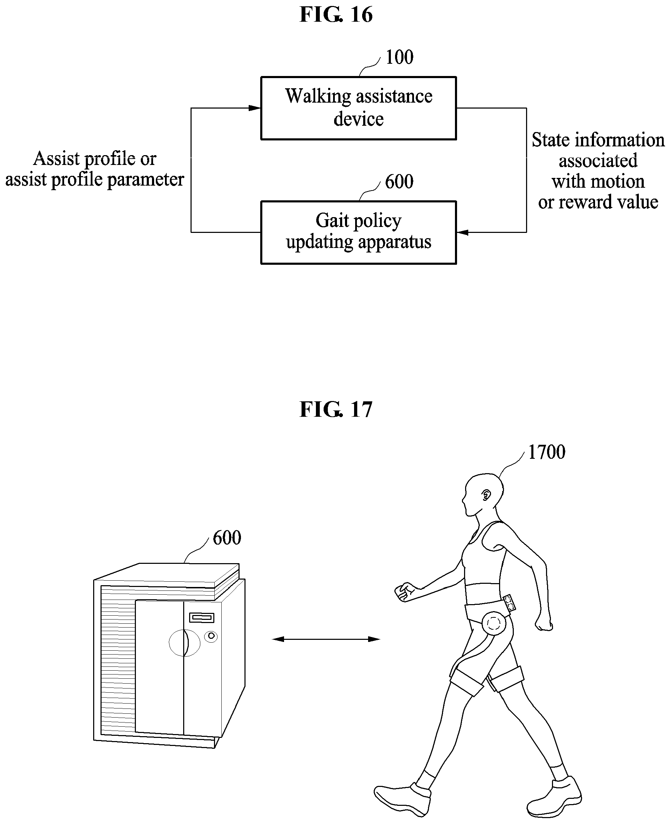

According to some example embodiments, the gait policy updating apparatus may be a server 1700 of FIG. 17, and may be a device that is physically separate from the walking assistance device 100. The personalized gait policy may be updated by the server 1700 and the updated personalized gait policy may be stored in the walking assistance device 100.

According to other example embodiments, the gait policy updating apparatus may be included in the walking assistance device 100. For example, the controller 300 may perform the functions of the gait policy updating apparatus such that operations 410 and 420 may be performed as a portion of a training of updating a gait policy improved (or, alternatively, optimized) for the user. That is, generating the assist profile based on the personalized gait policy and controlling the walking assistance device 100 based on the assist profile may be a portion of operations performed to update the gait policy that is improved (or, alternatively, optimized) for the user.

FIG. 5 is a graph showing an example of an assist torque profile according to at least one example embodiment.

The walking assistance device 100 may detect a current gait cycle based on state information associated with a motion of the walking assistance device 100. State information associated with the motion may include sensor values measured by the sensor 120, the IMU 130, and a pressure sensor (not shown) of the walking assistance device 100.

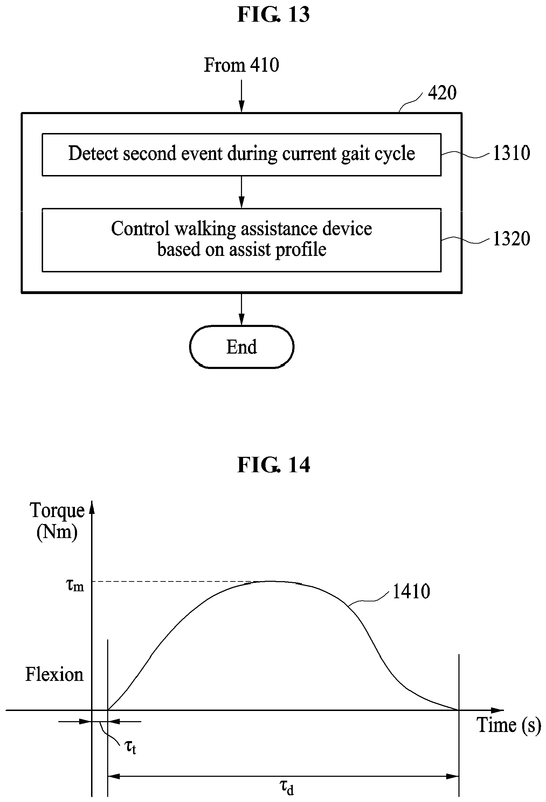

For clarity of description, a progress rate of a gait cycle is illustrates as 0% to 100%. However, the progress rate (n%) of the gait cycle may be converted to a time. For example, the progress rate 0% may be a second event point in time for a right leg and the progress rate 50% may be a second event point in time for a left leg, which is described with reference to FIG. 13. Also, the progress rate 100% may be a second event point in time for the right leg. That is, the assist torque profile may refer to an assist torque profile that is generated with respect to a single gait cycle of the right leg.

According to an example embodiment, an assist torque having a positive value may provide a force in a direction in which a leg moves from the front to the back. That is, if the user flexes the leg, the assist torque having the positive value may be output.

Conversely, an assist torque having a negative value may provide a force in which the leg moves from the back to the front. That is, if the user extends the leg, the assist torque having the negative value may be output.

The assist torque profile may be the assist torque profile for a left hip joint driver of the walking assistance device 100 and/or a right hip joint driver of the walking assistance device 100.

The assist torque profile is described above with FIG. 5. The description related thereto may be similarly applicable to the joint trajectory profile and the FES profile.

**<Personalized gait policy generation method>**

Hereinafter, a method of updating, by a gait policy updating apparatus, a personalized gait policy according to at least one example embodiment is described.

FIG. 6 is a diagram illustrating a configuration of a gait policy updating apparatus according to at least one example embodiment.

Referring to FIG. 6, a gait policy updating apparatus 600 includes a communicator 610, a processor 620, and a memory 630.

The communicator 610 is connected to the processor 620 and the memory 630 to transmit and receive data. The communicator 610 may be connected to an external device to transmit and receive data.

The communicator 610 may be configured as a circuitry within the gait policy updating apparatus 600. For example, the communicator 610 may include an internal bus and an external bus. As another example, the communicator 610 may be a component that connects the gait policy updating apparatus 600 and an external device. The communicator 610 may be an interface. The communicator 610 may receive data from the external device and may transmit data to the processor 620 and the memory 630.

The processor 620 processes data received by the communicator 610 and data stored in the memory 630. The processor 620 executes a computer-readable code, for example, software, stored in the memory 630 and instructions caused by the processor 620.

The memory 630 stores data received by the communicator 610 and data processed by the processor 620. For example, the memory 630 may store a program. The stored program may be a set of syntaxes that are coded to be capable of generating a personalized gait policy and to be executable by the processor 620.

According to an example embodiment, the memory 630 may include at least one volatile memory, non-volatile memory, RAM, a flash memory, a hard disk drive, and an optical disk drive.

The memory 630 stores an instruction set, for example, software, for operating the gait policy updating apparatus 600. The instruction set for operating the gait policy updating apparatus 600 is executed by the processor 620.

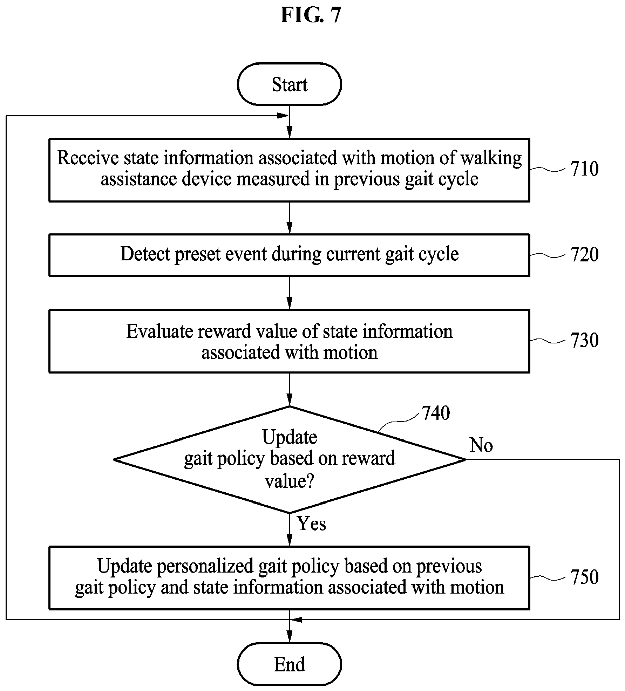

A further description related to the communicator 610, the processor 620, and the memory 630 will be made with reference to FIGS. 7 through 12.

FIG. 7 is a flowchart illustrating a gait policy updating method according to at least one example embodiment.

Operations 710 through 750 of FIG. 7 may be performed by the gait policy updating apparatus 600 of FIG. 6, and may be performed using an on-line scheme in which a user keeps walking with the walking assistance device 100 on and processes data about the walking in real time. The on-line scheme is distinguished from an off-line scheme of processing a gait policy using data collected after walking of the user is completed.

Referring to FIG. 7, in operation 710, the communicator 610 receives state information associated with a motion of the walking assistance device 100 measured in a previous gait cycle.

For example, the state information associated with the motion may include a change trajectory of a hip joint angle, a change trajectory of a knee joint angle, and/or a change trajectory of an ankle joint angle of the walking assistance device 100. A joint angle may be measured using the sensor 120 described above with FIGS. 1 and 2. The sensor 120 may measure the joint angle in real time and the communicator 610 may receive the measured joint angle in real time. That is, the communicator 610 may receive measurement values from at least one sensor of the walking assistance device 100 as state information associated with the motion of the walking assistance device 100.

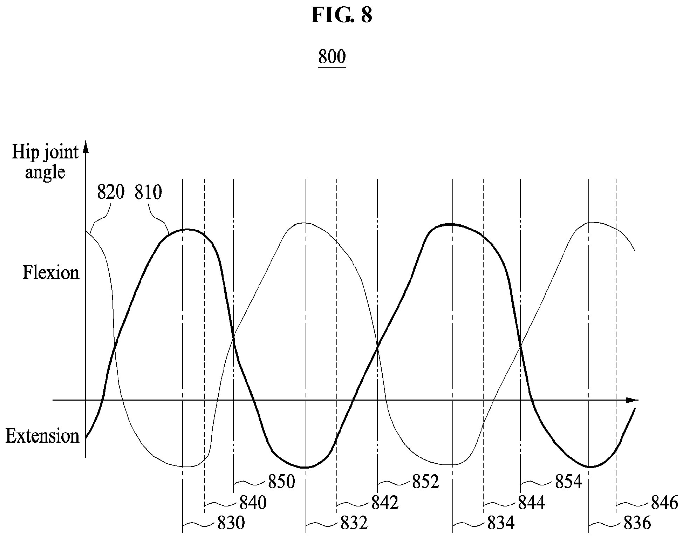

A change trajectory of an angle may be acquired by aligning measured joint angles based on a temporal axis. A further description related to the change trajectory of the angle will be made with reference to FIG. 8.

The processor 620 may generate additional state information based on the received state information associated with the motion of the walking assistance device 100. For example, the processor 620 may generate additional state information using the received measurement values, for example, a step duration, a swing time, a support time, a step length, and the like.

As another example, state information associated with the motion may include X axis, Y axis, and Z axis accelerations and X axis, Y axis, and Z axis angular velocities according to a walking motion of the user measured by the IMU 130 of the walking assistance device 100.

As another example, state information associated with the motion may include a sole pressure of the user measured by a pressure sensor (not shown) provided on a sole of the user.

In operation 720, the processor 620 detects the desired (or, alternatively, the preset) event during the current gait cycle. For example, the communicator 610 may consecutively receive state information associated with the motion of the walking assistance device 100, and the processor 620 may detect an event based on the state information associated with the motion of the walking assistance device 100 that is received in real time. The event may be detected based on measurement values received from the at least one sensor of the walking assistance device 100.

The event may be an event that a hip joint angle of a lead leg of the walking assistance device 100 is maximum. As another example, the event may be an event that both legs of the walking assistance device 100 cross. As another example, the event may be an event that the lead leg of the walking assistance device 100 is in contact with the ground. The processor 620 may detect the event that the lead leg is in contact with the ground based on a direction and a magnitude of an acceleration measured by the IMU 130 and a sole pressure measured by the pressure sensor. As another example, the event may be an event that a follow leg of the walking assistance device 100 leaves the ground. The processor 620 may determine a gait progress rate of a leg in real time and may detect the event if the determined progress rate corresponds to a preset progress rate.

If the desired (or, alternatively, a preset) event is detected, the processor 620 may classify a previous point in time as a previous gait cycle and a subsequent point in time as a current gait cycle based on a point in time at which the event is detected. Each of a gait cycle for a right leg and a gait cycle for a left leg may be defined to be different based on a corresponding reference leg.

For example, the event may be set for each of the left leg and the right leg. If the event for each of the left leg and the right leg is detected, a corresponding gait cycle may be restarted. For example, in the case of the event that the hip joint of the lead leg is maximum, an event in a case in which the left leg is the lead leg and an event in a case in which the right leg is the lead leg may be detected.

In operation 730, the processor 620 evaluates a reward value of state information associated with the motion of the walking assistance device 100. For example, an assist torque output based on a previous assist profile may be output to the user in a previous gait cycle. The reward value may be a value used to evaluate a result of a motion of the user and the walking assistance device 100 based on the assist torque output to the user in the previous gait cycle. The reward value may relate to state information associated with the motion of the walking assistance device 100 based on the previous assist profile that is generated based on the previous gait policy. Evaluating the reward value may indicate evaluating the gait policy.

According to an example embodiment, a reward value about the motion of the walking assistance device 100 may be calculated based on state information associated with the previous gait cycle. For example, a gait symmetry between the left leg and the right leg may be calculated as the reward value. The gait symmetry may be calculated according to Equation 1.

[Equation 1]

$$\text{Gait Symmery} = \frac{-2 \times |V_L - V_R|}{(V_L + V_R)}$$

In an example in which $V_L$ denotes a step duration of the left leg and $V_R$ denotes a step duration of the right leg in Equation 1, the step duration of the left leg and the step duration of the right leg may be calculated based on state information associated with the motion of the walking assistance device 100. According to Equation 1, if the step duration of the left leg and the step duration of the right leg are equal to each other, the calculated gait symmetry is zero. Otherwise, the calculated gait symmetry has a negative value.

In an example in which VL denotes a step length of the left leg and VR denotes a step length of the right leg in Equation 1, the step length of the left leg and the step length of the right leg may be calculated based on state information associated with the motion of the walking assistance device 100.

Although example embodiments of calculating the gait symmetry using the step duration and the step length are described using Equation 1, the gait symmetry may be calculated using a factor, such as a swing time and a stance time.

The reward value about the motion of the walking assistance device 100 may be calculated based on a power consumption amount of the walking assistance device 100. For example, the reward value may be calculated based on a power consumption amount for the driver 110 provided to the left leg and a power consumption about for the driver 110 provided to the right leg.

Also, the reward value about the motion of the walking assistance device 100 may be calculated based on a gait stability of the walking assistance device 100. The gait stability may be calculated based on a horizontal and vertical shaking level measured by the IMU 130.

Also, the reward value about the motion of the walking assistance device 100 may be calculated based on whether a corresponding gait is performed in a frontal direction of the user. Whether the gait is performed in the frontal direction may be determined based on data measured by the IMU 130. If supination or pronation is present in a hip joint or an ankle joint, the user may move to the side instead of moving in a complete frontal direction.

Also, the reward value about the motion of the walking assistance device 100 may be calculated based on metabolic cost.

Also, the reward value about the motion of the walking assistance device 100 may be received from the user of the walking assistance device 100 or a third party that evaluates the motion of the walking assistance device 100. For example, the third party may be a therapist that treats a patient wearing the walking assistance device 100. For example, the user or the therapist may transmit a signal indicating a discomfort level of the motion of the walking assistance device 100 to the gait policy updating apparatus 600 through a user interface. The user interface may include a button, a touch screen, and a microphone for voice recognition.

The processor 620 may calculate an average reward value based on a plurality of reward values that are evaluated over a desired (or, alternatively a predetermined) number of previous gait cycles. For example, the average reward value for previous three gait cycles may be calculated.

In operation 740, the processor 620 determines whether to update the gait policy based on the evaluated reward value. For example, if a motion of the left leg and a motion of the right leg perfectly match, the reward value calculated according to Equation 1 is zero. Although the motion of the left leg and the motion of the right leg do not perfectly match, the motion of the left leg and the motion of the right leg may have a similarity by a desired (or, alternatively, a predetermined) level. In this case, the motion of the left leg and the motion of the right leg may be evaluated to be symmetric to each other using a threshold. If the motion of the left leg and the motion of the right leg are not evaluated to be symmetric, the processor 620 may determine that the gait policy is to be updated.

As another example, if the reward value about the previous gait cycle has not changed within a desired (or, alternatively, a predetermined) range compared to a previous reward value, the processor 620 may determine that a previous gait policy is adjusted to be suitable for the user. In this case, the previous gait policy may be determined as a final gait policy. That is, if the reward value about the previous gait cycle has not changed within the range compared to the previous reward value, a process of updating the gait policy may be terminated.

If the reward value about the previous gait cycle differs from the previous reward value beyond the range, the processor 620 may determine that the previous gait policy is not adjusted to be suitable for the user. In this case, the processor the processor 620 may determine that the gait policy needs to be updated.

As another example, if the reward value is calculated a desired (or, alternatively,, a preset) number of times or less, the processor 620 may determine that the gait policy needs to be updated.

In operation 750, the processor 620 updates the personalized gait policy based on the previous gait policy and state information associated with the motion of the walking assistance device 100. For example, the gait policy may be configured using a neural network. The processor 620 may update the personalized gait policy by changing a weight of at least one parameter that constitutes the neural network. Updating the gait policy may indicate solving an optimization problem of maximizing the reward value about the motion of the walking assistance device 100. To update the gait policy, reinforcement learning may be applied. The processor 620 updates the gait policy to maximize a reward value to be evaluated with respect to a subsequent gait cycle.

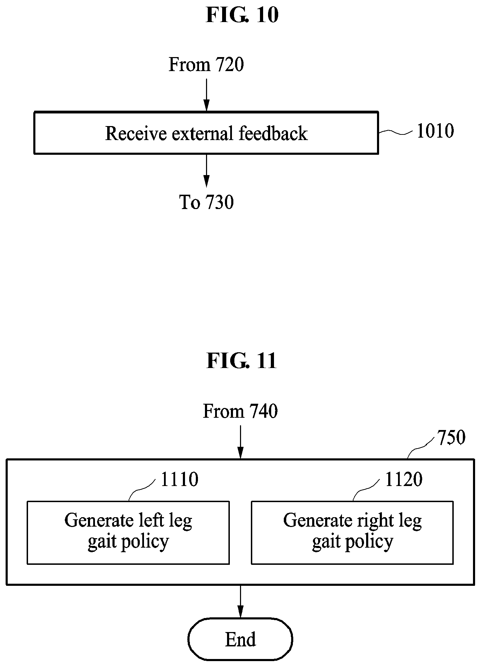

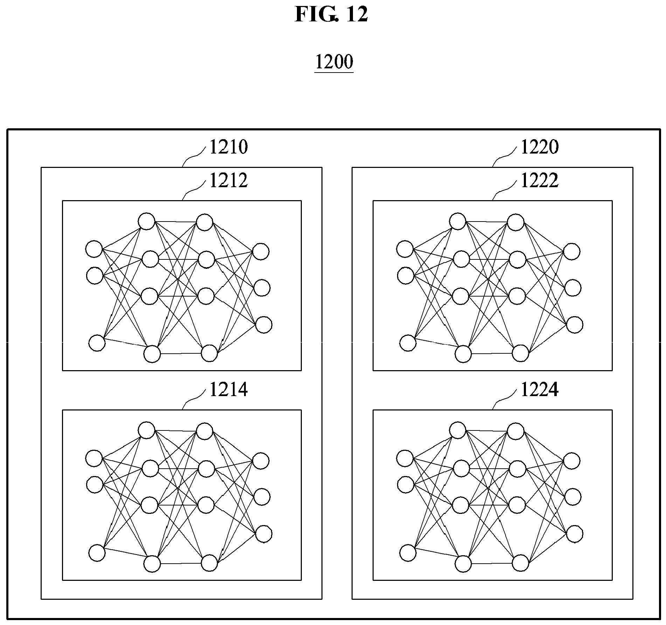

A method of updating a gait policy will be described with reference to FIGS. 11 and 12.

The method of updating the personalized gait policy by repeating operations 710 through 750 may be applied in a case in which the user keeps walking with wearing the walking assistance device 100. A plurality of gait cycles may be generated if the user keeps walking. A motion of a previous gait cycle is evaluated in a current gait cycle and a gait policy for a motion of the current gait cycle is updated based on an evaluation result. An assist profile for the current gait cycle is generated based on the updated gait policy and the motion of the current gait cycle occurs based on the assist profile. The above processes in which the motion of the current gait cycle becomes the motion of the previous gait cycle are repeated.

FIG. 8 is a graph showing an example of state information associated with a motion of a walking assistance device according to at least one example embodiment.

A hip joint angle trajectory 800 may be used as state information associated with a motion of the walking assistance device 100. The hip joint angle trajectory 800 includes a right hip joint angle trajectory 810 and a left hip joint angle trajectory 820.

The hip joint angle trajectory 800 relates to a plurality of gait cycles for description and a hip joint angle of the walking assistance device 100 is measured in real time. Accordingly, in an actual implementation, a hip joint angle trajectory of a future point in time of a measurement point in time of the hip joint angle is not acquired.

Points in times 830, 832, 834, and 836 each at which a hip joint angle of a lead leg is maximum may be detected from the right hip joint angle trajectory 810 and the left hip joint angle trajectory 820. At the points in times 830 and 834, the right hip joint angle may be maximum. At the points in times 832 and 836, the left hip joint angle may be maximum.

Points in times 840, 842, 844, and 846 each at which the lead leg is in contact with the ground may be detected from the right hip joint angle trajectory 810 and the left hip joint angle trajectory 820. At the points in times 840 and 844, the right leg may be in contact with the ground. At the points in times 842 and 846, the left leg may be in contact with the ground. The points in times 840, 842, 844, and 846 each at which the lead leg is in contact with the ground may be detected based on at least one of measurement values of the IMU 130 and the pressure sensor.

Points in times 850, 852, and 854 each at which both legs cross may be detected from the right hip joint angle trajectory 810 and the left hip joint angle trajectory 820.

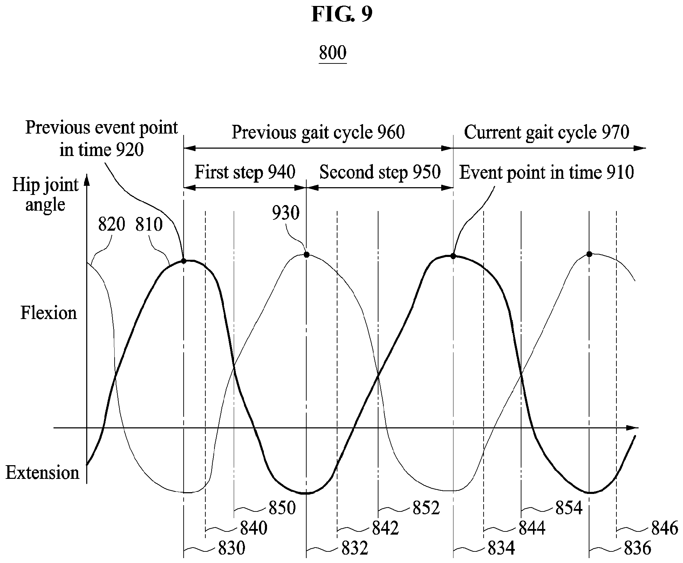

FIG. 9 is a graph showing an example of an event point in time detected based on state information associated with a motion of a walking assistance device according to at least one example embodiment.

An event that a hip joint angle of a lead leg is maximum may be preset as an event to be detected. A point in time at which the event is detected may be an event point in time.

For example, if a right leg is the lead leg, an event point in time 910 at which a right hip joint angle is maximum may be detected. If the event point in time 910 is detected, a following duration of the event point in time 910 may be defined as a current gait cycle 970. The current gait cycle 970 may be a duration from the event point in time 910 at which the event is detected to a point in time (not shown) at which a next event is detected. If an event for the right leg is detected in the current gait cycle 970, a next event corresponding to the detected event may be an event for the right leg detected in a subsequent gait cycle (not shown).

A duration from a previous event point in time 920 at which a previous event corresponding to the event is detected to the point in time 910 at which the event is detected may be defined as a previous gait cycle 960. If the event for the right leg is detected in the current gait cycle 970, the previous event corresponding to the detected event may be the event for the right leg detected in the previous gait cycle 960.

A single gait cycle may include two steps. That is, a single gait cycle may include a step of the left leg and a step of the right leg. For example, the previous gait cycle 960 may include a first step 940 that is the step of the left leg and a second step 950 that is the step of the right leg.

As another example, if the left leg is the lead leg, an event point in time, for example, the point in time 836, at which the left hip joint angle is maximum may be detected. If the point in time 836 is detected, a following duration of the point in time 836 may be defined as a current gait cycle.

A duration from a previous event point in time, for example, the point in time 832, at which the previous event corresponding to the event is detected to the point in time 836 at which the event is detected may be defined as a previous gait cycle. If the event for the left leg is detected in the current gait cycle, the previous event corresponding to the detected event may be the event for the left leg detected in the previous gait cycle.

A duration of the previous gait cycle and a duration of the current gait cycle for the left leg may differ from a duration of the previous gait cycle and a duration of the current gait cycle for the right leg. That is, a gait cycle may be defined for each of the left leg and the right leg.

FIG. 10 is a flowchart illustrating a method of receiving external feedback according to at least one example embodiment.

Operation 1010 of FIG. 10 may be performed after operation 720 of FIG. 7 is performed.

In operation 1010, the communicator 610 receives external feedback. The external feedback may be a reward value about a motion of the walking assistance device 100.

According to an example embodiment, the external feedback may be received from the user of the walking assistance device 100 and/or a third party that evaluates the motion of the walking assistance device 100. For example, the user or the therapist may transmit a signal indicating a discomfort level of the motion of the walking assistance device 100 to the gait policy updating apparatus 600 through a user interface. The user interface may include a button, a touch screen, and/or a microphone for voice recognition.

According to another example embodiment, the external feedback may be biosignal data of the user of the walking assistance device 100. For example, the biosignal data may be an EMG signal, an EEG signal, a heart rate, and/or metabolic data.

FIG. 11 is a flowchart illustrating a method of generating a personalized gait policy according to at least one example embodiment.

Operation 750 of FIG. 7 may include operations 1110 and 1120 of FIG. 11. Operations 1110 and 1120 may be performed in parallel. However, example embodiments are not limited thereto. For example, in some example embodiments, operations 1110 and 1120 may be performed sequentially in any order.

A gait capability of the left leg and a gait capability of the right leg of the user may differ from each other. For example, if the right leg of the user is paralyzed, a right leg gait policy and a left leg gait policy may differ from each other.

In operation 1110, the processor 620 generates a left leg gait policy for the left leg. For example, the processor 620 may generate a left flexion gait policy for a flexion mode of the left leg and may generate a left extension gait policy for an extension mode of the left leg. Although the flexion mode and the extension mode are described with respect to a motion of a leg, the description may be applied to an additional mode. In one example, a new gait policy may be generated by updating a previous gait policy.

The left flexion gait policy or the left extension gait policy may include policies for the respective portions of the left leg. For example, the left flexion gait policy may include a gait policy for a left hip joint, a gait policy for a left knee joint, and a gait policy for a left ankle joint. The gait policy for the left hip joint relates to a vertical motion, a horizontal motion, and supination/pronation of the left hip joint. The gait policy for the left ankle joint relates to a vertical motion and supination/pronation of the left ankle joint.

In operation 1120, the processor 620 generates a right leg gait policy for the right leg. For example, the processor 620 may generate a right flexion gait policy for a flexion mode of the right leg and a right extension gait policy for an extension mode of the right leg.

The right flexion gait policy or the right extension gait policy may include polices for the respective portions of the right leg. For example, the right flexion gait policy may include a gait policy for a right hip joint, a gait policy for a right knee joint, and a gait policy for a right ankle joint. The gait policy for the right hip joint relates to a vertical motion, a horizontal motion, and supination/pronation of the right hip joint. The gait policy for the right ankle joint relates to a vertical motion and supination/pronation of the right ankle joint.

FIG. 12 illustrates an example of a personalized gait policy according to at least one example embodiment.

A personalized gait policy 1200 may be configured as a neural network. The neural network may include a plurality of parameters. The neural network outputs a result by processing a received input based on the plurality of parameters. The personalized gait policy 1200 may receive, as an input, state information associated with a motion of the walking assistance device 100 that is measured in a previous gait cycle of a current point in time and may output a gait profile parameter to be used in a current gait cycle. If a weight of at least one of parameters of the neural network is changed, the gait profile parameter to be output may vary although the same input is received.

An assist profile parameter may be a factor used to generate an assist profile. For example, if the assist profile is an assist torque profile, the assist profile parameter may include parameters, such as a duration, a maximum torque, and an output timing of an assist torque. The assist profile may be generated using the assist profile parameter.

Referring to FIG. 12, the personalized gait policy 1200 includes a left leg gait policy 1210 and a right leg gait policy 1220. The left leg gait policy 1210 includes a left flexion gait policy 1212 and a left extension gait policy 1214. The right leg gait policy 1220 includes a right flexion gait policy 1222 and a right extension gait policy 1224.

The processor 620 may generate the personalized gait policy 1200 by simultaneously or sequentially generating four gait polices, for example, the left flexion gait policy 1212, the left extension gait policy 1214, the right flexion gait policy 1222, and the right extension gait policy 1224.

FIG. 13 is a flowchart illustrating a method of controlling a walking assistance device based on an assist profile according to at least one example embodiment.

Operation 420 of FIG. 4 may include operations 1310 and 1320 of FIG. 13.

Referring to FIG. 13, in operation 1310, the processor 320 detects a second event during a current gait cycle. The second event may be preset. The processor 320 may detect the second event based on state information associated with a motion of the walking assistance device 100 that is received in real time.

For example, the second event may be an event that a hip joint angle of a lead leg of the walking assistance device 100 is maximum. As another example, the second event may be an event that both legs of the walking assistance device 100 cross. As another example, the second event may be an event that the lead leg of the walking assistance device 100 is in contact with the ground. The processor 320 may detect the event that the lead leg is in contact with the ground based on a direction and a magnitude of acceleration measured by the IMU 130 and a sole pressure measured by the pressure sensor. As another example, the second event may be an event that a follow leg of the walking assistance device 100 leaves the ground.

The second event differs from the event (referred to as a first event to be distinguished from the second event) described above with FIGS. 7 through 9, and the second event lags behind the first event in terms of time. For example, if the first event is the event that the hip joint angle of the lead leg is maximum and a first event point in time is the point in time 834, the second event may be the event that the lead leg is in contact with the ground and a second event point in time may be the point in time 844.

In operation 1320, the processor 320 controls the walking assistance device 100 based on the assist profile.

For example, the assist profile may be the assist torque profile. The processor 320 may control the walking assistance device 100 so that the at least one driver 110 of the walking assistance device 100 may generate a torque based on the assist torque profile. For example, the processor 320 may generate a control signal corresponding to the assist torque profile and may transmit the generated control signal to the driver 110. The driver 110 may output an assist torque in response to the control signal.

As another example, the assist profile may be a functional electrical stimulation (FES) profile. The processor 320 may control the walking assistance device 100 so that a muscle stimulation device of the walking assistance device 100 may provide an FES to the user based on the FES profile. If the FES is applied to muscles of the user, the muscles of the user may contract, which may lead to generating muscular strength.

As another example, if minute artificial muscles are present in the body of the user, the processor 320 may control the walking assistance device 100 to stimulate the artificial muscles using an electrical stimulation or an electrical signal.

According to an example embodiment, operation 710 of FIG. 7 may be performed after operation 1320 is performed. That is, state information associated with the motion of the walking assistance device 100 acquired in operation 1320 is regarded as state information associated with the motion of the walking assistance device 100 that is measured in the previous gait cycle in operation 710.

FIG. 14 is a graph showing an example of an assist torque profile according to at least one example embodiment.

The processor 320 may control the walking assistance device 100 during a duration from a current second point in time of a current gait cycle to a next second event point in time corresponding to a current second event using an assist torque profile 1410. For example, if the current second event is detected for a right leg, a next second event may be detected for a left leg.

The assist torque profile 1410 may be generated based on an assist profile parameter that is generated based on the personalized gait policy 1200. Although FIG. 14 illustrates the assist torque profile 1410 for a flexion motion of a lead leg, the processor 320 may also generate an assist torque profile for an extension motion of a follow leg using the personalized gait policy 1200.

A trajectory of the assist torque profile 1410 may be generated based on an assist torque output timing $\tau t$, an assist torque duration $\tau d$, and a maximum assist torque $\tau m$. The assist torque output timing $\tau t$, the assist torque duration $\tau d$, and the maximum assist torque $\tau m$ may be assist profile parameters output based on the personalized gait policy 1200. If weights of parameters of the personalized gait policy 1200 are changed, the assist torque output timing $\tau t$, the assist torque duration $\tau d$, and the maximum assist torque $\tau m$ may be changed. If the assist torque output timing $\tau t$, the assist torque duration $\tau d$, and the maximum assist torque $\tau m$ are changed, the trajectory of the assist torque profile 1410 may be changed. That is, in response to updating the personalized gait policy 1200, the assist torque output timing $\tau t$, the assist torque duration $\tau d$, and the maximum assist torque $\tau m$ are adjusted.

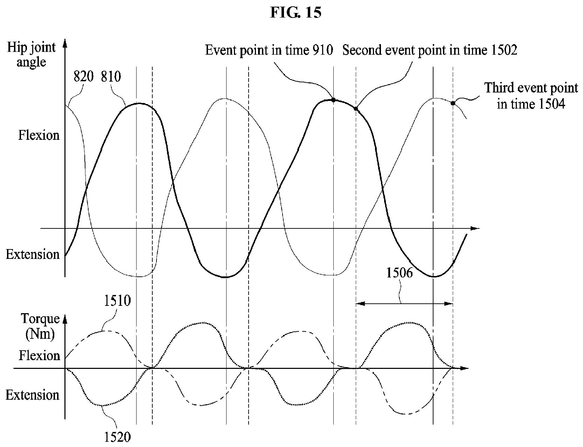

FIG. 15 is a graph showing an example of a trajectory of an assist profile according to at least one example embodiment.

Assist profiles of FIG. 15 are assist torque profiles.

Referring to FIG. 15, the right hip joint angle trajectory 810 may be caused by a right assist torque profile 1510 for a right leg and the left hip joint angle trajectory 820 may be caused by a left assist torque profile 1520 for a left leg.

An assist torque profile for a duration 1506 may be generated at the event point in time 910 at which the event is detected in real time. For example, a flexion assist torque profile for the right leg and an extension assist torque profile for the left leg may be generated. The flexion assist torque profile and the extension assist torque profile may be assist torque profiles for the duration 1506 from a second event point in time 1502 to a third event point in time 1504. If the second event point in time 1502 is a point in time at which an event that the right leg leaves the ground is detected, the third event point in time 1504 may be a point in time at which an event that the left leg leaves the ground is detected. A trajectory of a right hip joint and a trajectory of a left hip joint of the duration 1506 may be caused by the flexion assist torque profile and the extension assist torque profile.

FIG. 16 illustrates a relationship between a walking assistance device and a gait policy updating apparatus according to at least one example embodiment.

Referring to FIG. 16, a gait policy updating system includes the walking assistance device 100 and the gait policy updating apparatus 600.

The walking assistance device 100 is controlled based on a generated assist profile. State information associated with a motion occurring under control of the walking assistance device 100 and a reward value acquired by evaluating the state information are transmitted to the gait policy updating apparatus 600. State information associated with the motion may be consecutively received in real time and the reward value may be generated in response to detection of an event.

In response to receiving at least one of state information associated with the motion and the reward value, the gait policy updating apparatus 600 determines whether to update a gait policy used to generate the assist profile. For example, if the reward value is not received, the reward value may be calculated based on state information associated with the motion. If updating of the gait policy is required, the gait policy updating apparatus 600 updates the gait policy. For example, a neural network that constitutes the gait policy may be trained to maximize the reward value. The gait policy updating apparatus 600 generates an assist profile or an assist profile parameter that is used to control the walking assistance device 100 based on the updated gait policy, and transmits the generated assist profile or assist profile parameter to the walking assistance device 100.

The walking assistance device 100 is controlled based on the received assist profile or assist profile parameter. For example, if the assist profile parameter is received, the walking assistance device 100 generates the assist profile based on the assist profile parameter. If an event is detected, the walking assistance device 100 is controlled based on the assist profile. The walking assistance device 100 may measure state information associated with a motion occurring under the control.

FIG. 17 illustrates a gait policy updating apparatus configured as a server according to at least one example embodiment.

According to an example embodiment, the gait policy updating apparatus 600 may be configured in a form of a server. The gait policy updating apparatus 600 configured as the server may be a cloud server or may be connected to a plurality of walking assistance devices in a wireless or wired manner. A walking assistance device 1700 may correspond to the walking assistance device 100 of FIGS. 1 and 2.

Operation 410 of FIG. 4 may be performed by the gait policy updating apparatus 600. The gait policy updating apparatus 600 receives state information associated with a motion of the walking assistance device 1700 from the walking assistance device 1700 in real time. The gait policy updating apparatus 600 generates an assist profile or an assist profile parameter based on a personalized gait assist policy for the walking assistance device 1700. The generated assist profile or assist profile parameter may be transmitted to the walking assistance device 1700.

Once the walking assistance device 1700 receives the assist profile or the assist profile parameter, operation 420 of FIG. 4 may be performed. For example, when the walking assistance device 1700 receives the assist profile, the walking assistance device 1700 may be controlled based on the assist profile. As another example, when the walking assistance device 1700 receives the assist profile parameter, the walking assistance device 1700 may generate the assist profile based on the assist profile parameter and the walking assistance device 1700 may be controlled based on the generated assist profile.

If a user of the walking assistance device 1700 is walking, operations 710 through 750 of FIG. 7 and operations 410 and 420 of FIG. 4 may be repeatedly performed.

The gait policy updating apparatus 600 and the walking assistance device 1700 may exchange data through wired or wireless communication. For example, the walking assistance device 1700 may transmit, to the gait policy updating apparatus 600, state information associated with a motion of the walking assistance device 1700 that is measured in real time. The gait policy updating apparatus 600 may receive state information associated with the motion of the walking assistance device 1700 about a previous gait cycle. The gait policy updating apparatus 600 may detect a preset event based on the state information and may evaluate a gait policy based on the state information in response to an occurrence of the event. The gait policy updating apparatus 600 may update the gait policy based on the evaluation and state information associated with the motion of the walking assistance device 1700. The gait policy updating apparatus 600 may automatically update the gait policy during walking of the walking assistance device 1700. That is, if the user keeps walking, the gait policy may be automatically updated during the walking.

The gait policy updating apparatus 600 may transmit the finally updated personalized gait policy to the walking assistance device 1700.

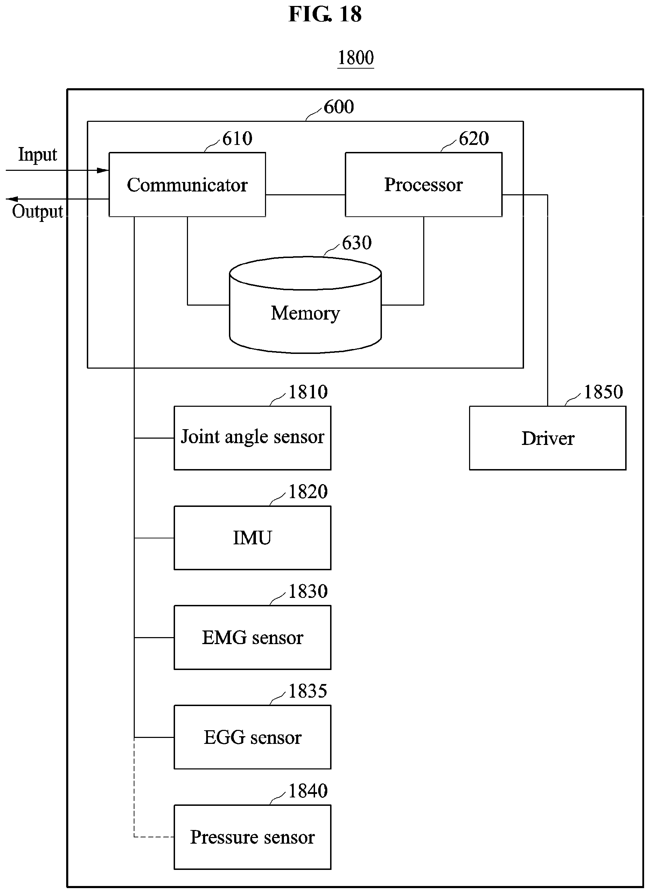

FIG. 18 is a diagram illustrating a gait policy updating apparatus included in a walking assistance device according to at least one example embodiment.

According to another example embodiment, the gait policy updating apparatus 600 may be included in a walking assistance device 1800. For example, the gait policy updating apparatus 600 may include the controller 300 described above with FIGS. 3 and 4. The processor 620 of the gait policy updating apparatus 600 may perform the same functions as those of the processor 320 of the controller 300. As another example, the walking assistance device 1800 may control the controller 300 and the gait policy updating apparatus 600 using a plurality of processors.

A joint angle sensor 1810 may measure a joint angle of a user or an angle of a driver 1850 that is provided around a joint of the user. For example, the joint angle sensor 1810 may measure a hip joint angle, a knee joint angle, and an ankle joint angle.

An IMU 1820 may correspond to the IMU 130 described above with FIGS. 1 and 2.

An EMG sensor 1830 may measure an electrical signal occurring in muscles of the user. A measured EMG signal may be one of the external feedbacks described above with FIG. 10. That is, the EMG signal may be used to evaluate a reward value.

An EGG sensor 1835 may measure an electrical signal occurring in the brain of the user. A measured EGG signal may be one of the external feedbacks described above with FIG. 10. That is, the EGG signal may be used to evaluate a reward value.

A pressure sensor 1840 is provided on a sole of the user and may measure a pressure of the sole. For example, the pressure sensor 1840 may transmit a wirelessly measured pressure to the communicator 610. The processor 620 may detect an event that a lead leg is in contact with the ground based on the measured pressure.

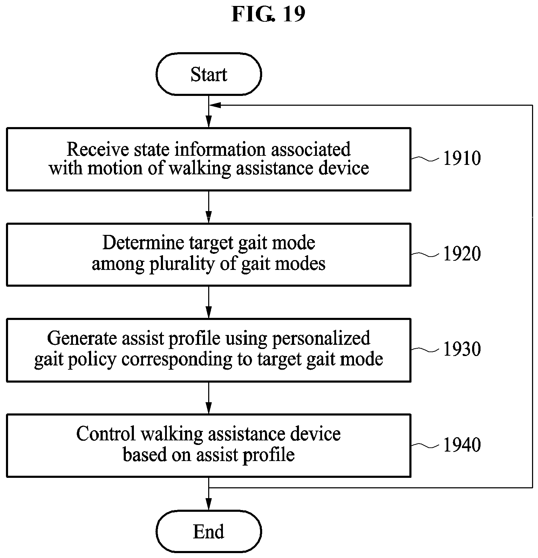

FIG. 19 is a flowchart illustrating a method of controlling a walking assistance device based on a determined target mode according to at least one example embodiment.

Operations 1910 through 1940 may be performed by the walking assistance device 100 described above with FIGS. 1 through 5. Operations 1910 through 1940 may be performed in a walking situation in which the walking assistance device 100 is controlled based on a personalized gait policy. That is, operations 1910 through 1940 may be performed in a situation in which personalization of the walking assistance device 100 is completed and the user is using the walking assistance device 100 in real life.

Although operations 1910 through 1940 are described to be performed by the controller 300, operations 1910 through 1940 may be performed by the walking assistance device 1700 of FIG. 17 or by the walking assistance device 1800 of FIG. 18.

Referring to FIG. 19, in operation 1910, the communicator 310 receives state information associated with a motion of the walking assistance device 100. The communicator 310 receives state information associated with the motion of the walking assistance device 100 that is measured in a previous gait cycle.

For example, state information associated with the motion may include a change trajectory of a hip joint angle, a change trajectory of a knee joint angle, and a change trajectory of an ankle joint angle of the walking assistance device 100.

In operation 1920, the processor 320 determines a target gait mode among a plurality of gait modes. For example, the processor 320 may determine the target gait mode based on state information associated with the motion of the walking assistance device 100. The plurality of gait modes may include a flatland walking mode, a slope-ascending mode, a slope-descending mode, a step-ascending mode, a step-descending mode, and a running mode.

In operation 1930, the processor 320 generates an assist profile using a personalized gait policy corresponding to the target gait mode. For example, a personalized gait policy may be generated in advance for each gait mode. The processor 320 may generate the assist profile based on the determined personalized gait policy.

In operation 1940, the processor 320 controls the walking assistance device 100 based on the generated assist profile.

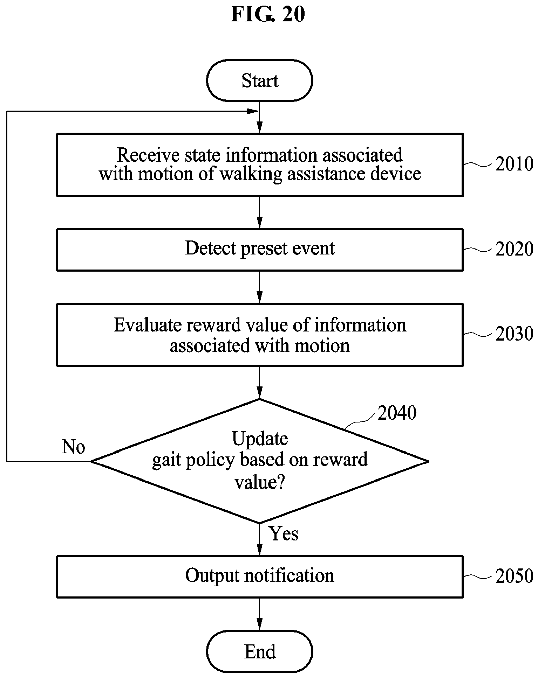

FIG. 20 is a flowchart illustrating a method of outputting a notification of requesting updating of a personalized gait policy according to at least one example embodiment.

Operations 2010 through 2050 may be performed by the walking assistance device 100 described above with FIGS. 1 through 5. Operations 2010 through 2050 may be performed in a walking situation in which the walking assistance device 100 is controlled based on the personalized gait policy. For example, in response to a change in a walking habit of a user, operations 2010 through 2050 may be performed.

Although operations 2010 through 2050 are described to be performed by the controller 300, operations 2010 through 2050 may be performed by the walking assistance device 1700 of FIG. 17 or by the walking assistance device 1800 of FIG. 18.

Referring to FIG. 20, in operation 2010, the communicator 310 receives state information associated with a motion of the walking assistance device 100.

In operation 2020, the processor 320 detects a desired (or, alternatively, a preset) event based on state information associated with the motion of the walking assistance device 100.

In operation 2030, the processor 320 evaluates a reward value of state information associated the motion of the walking assistance device 100.

In operation 2040, the processor 320 determines whether to update a gait policy based on the evaluated reward value.

In operation 2050, if updating of the gait policy is determined to be required, the processor 320 outputs a notification that requests the user to update the gait policy. Once the notification is output, the user may perform a procedure of updating the gait policy. For example, operations 710 through 750 of FIG. 7 may be performed to update the gait policy.

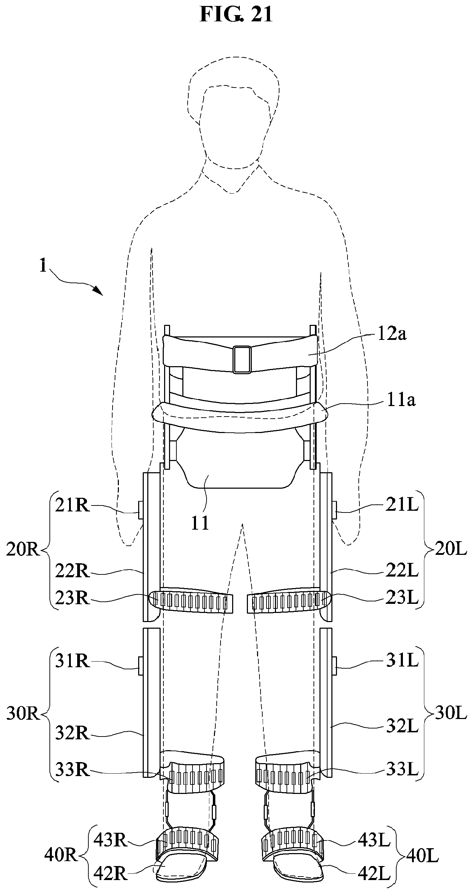

FIGS. 21 through 23 illustrate examples of a walking assistance device according to at least one example embodiment.

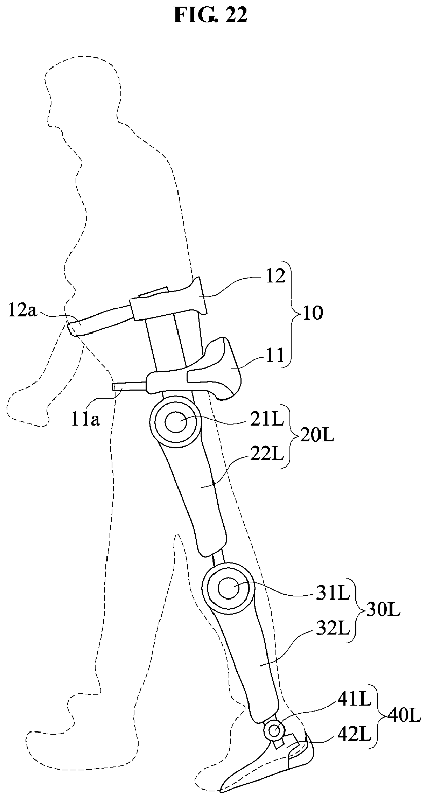

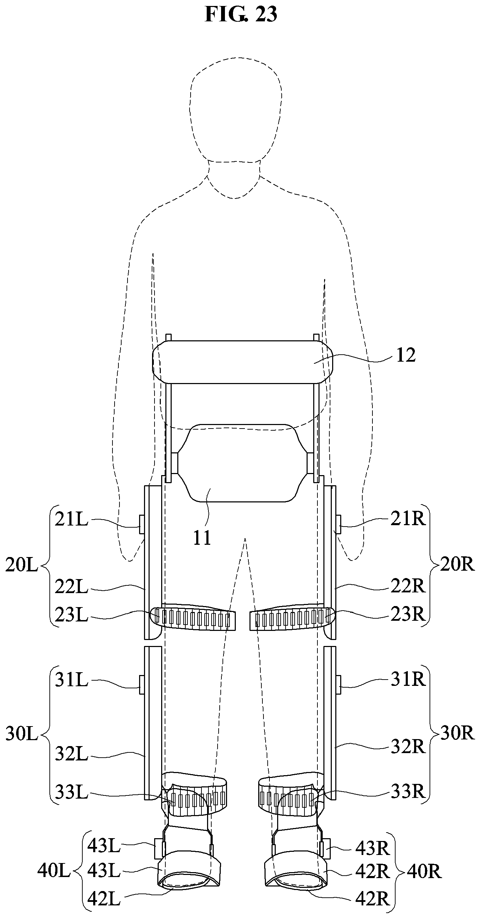

FIGS. 21 through 23 illustrate examples of a walking assistance device 1 applicable to a human body. FIG. 21 is a front view of the walking assistance device 1, FIG. 22 is a side view of the walking assistance device 1, and FIG. 23 is a rear view of the walking assistance device 1.

The walking assistance device 1 may include the driver 110, the sensor 120, the IMU 130, and the controller 140, which are described above.

Referring to FIGS. 21 through 23, the walking assistance device 1 is in an exoskeleton structure to be wearable to each of a left leg and a right leg of a user. The user may perform a motion, for example, an extension motion, a flexion motion, an adduction motion, and an abduction motion, with wearing the walking assistance device 1. The extension motion is a movement that extends a joint, and the flexion motion is a movement that flexes a joint. The adduction motion is a movement that moves a leg to be close to a central axis of the body, and the abduction motion is a movement that extends a leg to be away from the central axis of the body.

Referring to FIGS. 21 through 23, the walking assistance device 1 may include a body 10 and a mechanical part, for example, first structural parts 20R and 20L, second structural parts 30R and 30L, and third structural parts 40R and 40L.

The body 10 may include a housing 11. Various parts may be embedded in the housing 11. The parts embedded in the housing 11 may include, for example, a central processing unit (CPU), a printed circuit board (PCB), various types of storage devices, and a power source. For example, the body 10 may include the controller 140. The controller 140 may include the CPU and the PCB.

The CPU may be a microprocessor. The microprocessor may include an arithmetic logic operator, a register, a program counter, a command decoder and/or a control circuit in a silicon chip. The CPU may generate a control mode suitable for a walking environment, and may generate a control signal for controlling an operation of a mechanical part based on the selected control mode.

The PCB refers to a board on which a predetermined circuit is printed and may include the CPU and/or various storage devices. The PCB may be fixed in the housing 11.

Various types of storage devices may be included in the housing 11. The storage devices may include a magnetic disk storage device to store data by magnetizing the surface of a magnetic disk and a semiconductor memory device to store data using various types of memory semiconductors.

The power source embedded in the housing 11 may supply power to various types of parts embedded in the housing 11 or the mechanical part, for example, the first structural parts 20R and 20L, the second structural parts 30R and 30L, and the third structural parts 40R and 40L.

The body 10 may further include a waist support 12 configured to support a waist of the user. The waist support 12 may be in a shape of a curved flat plate to support the waist of the user.

The body 10 may further include a fastener 11a configured to fasten the housing 11 to a hip portion of the user and a fastener 12a configured to fasten the waist support 12 to the waist of the user. The fastener 11a, 12a may be configured as one of a band, a belt, and a strap having elasticity.

The body 10 may include the IMU 130. For example, the IMU 130 may be provided outside or inside the housing 11. The IMU 130 may be installed on the PCB embedded in the housing 11. The IMU 130 may measure an acceleration and an angular velocity.

Referring to FIGS. 21 through 23, the mechanical part may include the first structural part 20R, 20L, the second structural part 30R, 30L, and the third structural part 40R, 40L

The first structural part 20R, 20L may assist a motion of a femoral region and a hip joint of the user during a gait operation. The first structural parts 20R and 20L may include first drivers 21R and 21L, first supports 22R and 22L, and first fasteners 23R and 23L, respectively.

The driver 110 may include the first driver 21R, 21L. The description related to the driver 110 made with reference to FIGS. 1 through 20 may be applied to the first driver 21R, 21L.

The first driver 21R, 21L may be provided at a location of a corresponding hip joint of the first structural part 20R, 20L, and may generate a rotational force in a predetermined direction at various magnitudes. The rotational force generated by the first driver 21R, 21L may be applied to the first support 22R, 22L. The first driver 21R, 21L may be set to rotate within the movement range of a hip joint of the human body.

The first driver 21R, 21L may be driven in response to a control signal provided from the body 10. Although the first driver 21R, 21L may be configured as one of a motor, a vacuum pump, and a hydraulic pump, it is provided as an example only.

A joint angle sensor may be installed around the first driver 21R, 21L. The joint angle sensor may detect an angle at which the first driver 21R, 21L rotates based on a rotational axis. The sensor 120 may include the joint angle sensor.

The first support 22R, 22L may be physically connected to the first driver 21R, 21L. The first support 22R, 22L may rotate in a predetermined direction based on the rotational force generated by the first driver 21R, 21L.

The first support 22R, 22L may be provided in various shapes. For example, the first support 22R, 22L may be in a shape in which a plurality of knuckles are inter-connected. Here, a joint may be provided between the knuckles. The first support 22R, 22L may bend within a predetermined range by the joint. As another example, the first support 22R, 22L may be provided in a bar shape. Here, the first support 22R, 22L may be configured using a flexible material to be bendable within a predetermined range.

The first fastener 23R, 23L may be provided to the first support 22R, 22L. The first fastener 23R, 23L serves to fasten the first support 22R, 22L to a corresponding femoral region of the user.

FIGS. 21 through 23 illustrate an example in which the first supports 22R and 22L are fastened to the outside of the femoral regions of the user by the first fasteners 23R and 23L, respectively. If the first support 22R, 22L rotates in response to driving of the first driver 21R, 21L, the femoral region to which the first support 22R, 22L is fastened may rotate in the same direction in which the first support 22R, 22L rotates.

The first fastener 23R, 23L may be configured as one of a band, a belt, and a strap having elasticity, or may be configured using a metal material. FIG. 21 illustrates an example in which the first fastener 23R, 23L is configured using a chain.

The second structural part 30R, 30L may assist a motion of a lower leg and a knee joint of the user during a gait operation. The second structural parts 30R and 30L include second drivers 31R and 31L, second supports 32R and 32L, and second fasteners 33R and 33L, respectively.

The second driver 31R, 31L may be provided at a location of a corresponding knee joint of the second structural part 30R, 30L, and may generate a rotational force in a predetermined direction at various magnitudes. The rotational force generated by the second driver 31R, 31L may be applied to the second support 22R, 22L. The second driver 31R, 31L may be set to rotate within a movement range of a knee joint of the human body.

The driver 110 may include the second driver 31R, 31L. The description related to the hip joint made with reference to FIGS. 1 through 20 may be similarly applied to the knee joint.

The second driver 31R, 31L may be driven in response to a control signal provided from the body 10. Although the second driver 31R, 31L may be configured as one of a motor, a vacuum pump and a hydraulic pump, it is provided as an example only.

A joint angle sensor may be installed around the second driver 31R, 31L. The joint angle sensor may detect an angle at which the second driver 31R, 31L rotates based on a rotational axis. The sensor 120 may include the joint angle sensor.

The second support 32R, 32L may be physically connected to the second driver 31R, 31L. The second support 32R, 32L may rotate in a predetermined direction based on the rotational force generated by the second driver 31R, 31L.

The second fastener 33R, 33L may be provided to the second support 32R, 32L. The second fastener 33R, 33L serves to fasten the second support 32R, 32L to a lower leg portion of the user. FIGS. 21 through 23 illustrate an example in which the second supports 32R and 32L are fastened at the outside of lower leg portions of the user by the second fasteners 33R and 33L, respectively. If the second support 33R, 33L rotates in response to driving of the second driver 31R, 31L, the lower leg portion to which the second support 33R, 33L is fastened may rotate in the same direction in which the second support 33R, 33L rotates.

The second fastener 33R, 33L may be configured as one of a band, a belt, and a strap having elasticity, or may be configured using a metal material.

The third structural part 40R, 40L may assist a motion of an ankle joint and related muscles of the user during a gait operation. The third structural parts 40R and 40L may include third drivers 41R and 41L, foot supports 42R and 42L, and third fasteners 43R and 43L, respectively.

The driver 110 may include the third driver 41R, 41L. The description related to the hip joint made with reference to FIGS. 1 through 20 may be similarly applied to the ankle joint.

The third driver 41R, 41L may be provided to a corresponding ankle joint of the third structural part 40R, 40L, and may be driven in response to a control signal provided from the body 10. Similar to the first driver 21R, 21L or the second driver 31R, 31L, the third driver 41R, 41L may be configured as a motor.

A joint angle sensor may be installed around the third driver 41R, 41L. The joint angle sensor may detect an angle at which the third driver 41R, 41L rotates based on a rotational axis. The sensor 120 may include the joint angle sensor.

The foot support 42R, 42L may be provided at a location corresponding to a sole of the user, and may be physically connected to the third driver 41R and 41L.

A pressure sensor configured to detect a weight of the user may be provided to the foot support 42R, 42L. A detection result of the pressure sensor may be used to determine whether the user is wearing the walking assistance device 1, whether the user stands, whether a foot of the user is in contact with the ground, and the like.

The third fastener 43R, 43L may be provided to the foot support 42R, 42L. The third fastener 43R, 43L serves to fasten a foot of the user to the foot support 42R, 42L.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

The software may include a computer program, a piece of code, an instruction, or some combination thereof, to independently or collectively instruct and/or configure the processing device to operate as desired, thereby transforming the processing device into a special purpose processor. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

Example embodiments of the inventive concepts having thus been described, it will be obvious that the same may be varied in many ways. Such variations are not to be regarded as a departure from the intended scope of example embodiments of the inventive concepts, and all such modifications as would be obvious to one skilled in the art are intended to be included within the scope of the following claims.

**Claims**

1.  A method of controlling a walking assistance device, the method comprising:

    generating an assist profile for controlling the walking assistance device during a current gait cycle based on a personalized gait policy, the personalized gait policy being generated by,

    > receiving state information associated with a motion of the walking assistance device measured in a previous gait cycle, the previous gait cycle being a gait cycle occurring before the current gait cycle,
    > detecting a first event during the current gait cycle,
    > evaluating a reward value of state information associated with the motion at a point in time at which the first event is detected, and
    > selectively updating the personalized gait policy based on the state information associated with the motion and the reward value; and

    controlling the walking assistance device based on the assist profile.

2.  The method of claim 1, wherein the state information associated with the motion includes a change trajectory of one or more of a hip joint angle, a knee joint angle, and an ankle joint angle of the walking assistance device and/or wherein the detecting the first event comprises detecting one of:

    > an event that a hip joint angle of a lead leg of the walking assistance device is maximum;
    > an event that both legs of the walking assistance device cross;
    > an event that the lead leg of the walking assistance device is in contact with a ground; and

    an event that a follow leg of the walking assistance device leaves the ground and/or, wherein
    the previous gait cycle is from a point in time at which a previous event corresponding to the first event is detected to a point in time at which the first event is detected, and
    the current gait cycle is from the point in time at which the first event is detected to a point in time at which a next event corresponding to the first event is detected, and/or
    wherein the generating of the assist profile comprises:

    > generating the assist profile based on the state information associated with the motion and the personalized gait policy, and/or
    > wherein the receiving of the state information comprises:

    >> receiving measurement values from at least one sensor of the walking assistance device, the measurement values being the state information associated with the motion, wherein
    >> the detecting the first event detects the first event based on the measurement values, and/or
    >> further comprising generating, by the walking assistance device, the personalized gait policy.

3.  The method of claim 1 or 2, wherein the reward value relates to state information about the motion based on a previous assist profile generated based on a previous gait policy, the previous gait policy being a gait policy in effect prior to the personalized gait policy, wherein the updating of the personalized gait policy preferably comprises:
    updating a neural network of the personalized gait policy by changing a weight of a parameter that constitutes the neural network of the previous gait policy.

4.  The method of any of claims 1-3, wherein the updating of the personalized gait policy comprises:

updating a left-leg gait policy for a left leg of the walking assistance device; and
updating a right-leg gait policy for a right leg of the walking assistance device, wherein the updating of the left-leg gait policy preferably comprises at least one of:

updating a left-flexion gait policy for a flexion mode of the left leg of the walking assistance device; and
updating a left-extension gait policy for an extension mode of the left leg of the walking assistance device.

5. The method of any of claims 1-4, wherein the controlling of the walking assistance device comprises:

detecting a second event during the current gait cycle; and
controlling, from a point in time at which the second event is detected, the walking assistance device to operate based on the assist profile.

6. The method of claim 5, wherein the controlling of the walking assistance device comprises:
instructing at least one driver of the walking assistance device to generate a torque based on the assist profile, and/or wherein the controlling of the walking assistance device comprises:
providing a functional electrical stimulus to a user based on the assist profile.

7. The method of any of claims 1-6, wherein the reward value includes one or more of a gait symmetry of state information associated with the motion, a power consumption amount of state information associated with the motion, a gait stability of state information associated with the motion, metabolic cost, and external feedback, wherein the walking assistance device is preferably configured to calculate the gait symmetry based on one or more of a step length and a step duration of a user.

8. The method of any of claims 1-7, wherein the walking assistance device is configured to communicate with a server, and the updating the personalized gait policy comprises:
updating, by the server, update the personalized gait policy based on the state information.

9. The method of claim 8, further comprising:
receiving, by the walking assistance device, the personalized gait policy from the server, and/or wherein the controlling of the walking assistance device comprises:
transmitting, by the server, the assist profile to the walking assistance device, and/or
wherein the controlling of the walking assistance device comprises:

generating, by the server, a control signal for controlling a driver of the walking assistance device based on the assistance profile; and
transmitting the control signal to the walking assistance device to control the walking assistance device.

10. A method of updating a personalized gait policy to control a walking assistance device, preferably used as the selectively updating the personalized gait policy based on the state information associated with the motion and the reward value of any of the previous claims, the method comprising:

receiving state information associated with a motion of the walking assistance device measured in a previous gait cycle;
detecting an event during a current gait cycle;
evaluating a reward value of state information associated with the motion at a point in time at which the event is detected;
selectively updating the personalized gait policy based on the state information associated with the motion and the reward value;
generating an assist profile or an assist profile parameter based on the personalized gait policy; and
transmitting, to the walking assistance device, the assist profile or the assist profile parameter to control the walking assistance device.

11. A method of updating a personalized gait policy to control a walking assistance device during a current gait cycle, preferably used as the selectively updating the personalized gait policy based on the state information associated with the motion and the reward value of any claims 1-10, the method comprising:

receiving state information of the walking assistance device about a previous gait cycle, the previous gait cycle being a gait cycle occurring before the current gait cycle;

evaluating the personalized gait policy based on the state information in response to an occurrence of an event; and

selectively updating the gait policy during a walking of the walking assistance device based on the evaluation, wherein the evaluating of the personalized gait policy preferably comprises evaluating the personalized gait policy based on one or more of a gait symmetry, a power consumption amount, a gait stability, metabolic energy, and external feedback, and wherein the method is preferably performed by a server, the server configured to communicate with the walking assistance device.

12. A server configured to update a personalized gait policy to control a walking assistance device during a current gait cycle, the server comprising:

a memory configured to store a program to control the walking assistance device; and
a processor configured to execute the program to,

receive state information associated with a motion of the walking assistance device measured in a previous gait cycle, the previous gait cycle being a gait cycle occurring before the current gait cycle,
detect an event during the current gait cycle,
evaluate a reward value of state information associated with the motion at a point in time at which the event is detected;
selectively update the personalized gait policy based on the state information associated with the motion and the reward value;
generate an assist profile or an assist profile parameter based on the personalized gait policy; and
transmit, to the walking assistance device, the assist profile or the assist profile parameter to control the walking assistance device.

13. A non-transitory computer-readable medium storing computer readable instructions, which when executed by a computer, configure the computer to perform the method of any of claims 1-11.

14. A walking assistance device comprising:

a memory configured to store a program for controlling the walking assistance device; and
a processor configured to execute the program to,

generate an assist profile to control the walking assistance device during a current gait cycle based on a personalized gait policy, the personalized gait policy being generated by,

receiving state information associated with a motion of the walking assistance device measured in a previous gait cycle, the previous gait cycle being a gait cycle occurring before the current gait cycle,
detecting a first event during the current gait cycle,
evaluating a reward value of state information associated with the motion at a point in time at which the first event is detected, and
selectively updating the personalized gait policy based on the state information associated with the motion and the reward value; and

control the walking assistance device based on the assist profile.

15. The walking assistance device of claim 14, wherein the processor of the walking assistance device is configured to update the personalized gait policy, and/or wherein the walking assistance device is configured to,

receive the personalized gait policy from a server, the server being configured to update the personalized gait policy.

## FIG. 1

# FIG. 2

**FIG. 3**

300

Input →

Output ←

| ~310 Communicator |
| ~320 Processor |
| ~330 Memory |

**FIG. 4**

Start

Generate assist profile based on personalized gait policy — 410

Control walking assistance device based on assist profile — 420

End

**FIG. 5**

**FIG. 6**

600

**FIG. 7**

```
                    ┌─────────────┐
                    │    Start    │
                    └─────────────┘
                          │
    ┌─────────────────────┤
    │   ┌──────────────────────────────────────────────────┐
    │   │ Receive state information associated with motion   │
    │   │ of walking assistance device measured in previous  │──── 710
    │   │ gait cycle                                         │
    │   └──────────────────────────────────────────────────┘
    │                     │
    │   ┌──────────────────────────────────────────────────┐
    │   │ Detect preset event during current gait cycle      │──── 720
    │   └──────────────────────────────────────────────────┘
    │                     │
    │   ┌──────────────────────────────────────────────────┐
    │   │ Evaluate reward value of state information         │──── 730
    │   │ associated with motion                             │
    │   └──────────────────────────────────────────────────┘
    │                     │
    │                ╱────────────╲        740
    │              ╱   Update       ╲     No
    │            ╱  gait policy based  ╲───────────┐
    │            ╲  on reward value?   ╱           │
    │              ╲                 ╱             │
    │                ╲────────────╱               │
    │                     │ Yes                   │
    │   ┌──────────────────────────────────────┐ │
    │   │ Update personalized gait policy based  │ │
    │   │ on previous gait policy and state      │─── 750
    │   │ information associated with motion     │ │
    │   └──────────────────────────────────────┘ │
    │                     │◄──────────────────────┘
    └─────────────────────┤
                          │
                    ┌─────────────┐
                    │     End     │
                    └─────────────┘
```

**FIG. 8**

800

# FIG. 9

<u>800</u>

## FIG. 10

From 720

Receive external feedback — 1010

To 730

## FIG. 11

From 740

750

1110

Generate left leg
gait policy

1120

Generate right leg
gait policy

End

# FIG. 12

<u>1200</u>

**FIG. 13**

From 410

420

Detect second event during current gait cycle — 1310

Control walking assistance device based on assist profile — 1320

End

**FIG. 14**

Torque (Nm)

$\tau_m$

1410

Flexion

$\tau_t$

$\tau_d$

Time (s)

FIG. 15

## FIG. 16

100

Walking assistance device

Assist profile or assist profile parameter

State information associated with motion or reward value

600

Gait policy updating apparatus

## FIG. 17

1700

600

## FIG. 18

1800

## FIG. 19

```
         ┌─────────┐
         │  Start  │
         └─────────┘
              │
              ▼
┌────────────────────────────────────┐
│  Receive state information associated │ ∼ 1910
│  with motion of walking assistance device │
└────────────────────────────────────┘
              │
              ▼
┌────────────────────────────────────┐
│       Determine target gait mode      │ ∼ 1920
│       among plurality of gait modes   │
└────────────────────────────────────┘
              │
              ▼
┌────────────────────────────────────┐
│  Generate assist profile using personalized │ ∼ 1930
│  gait policy corresponding to target gait mode │
└────────────────────────────────────┘
              │
              ▼
┌────────────────────────────────────┐
│     Control walking assistance device  │ ∼ 1940
│     based on assist profile           │
└────────────────────────────────────┘
              │
              ▼
         ┌─────────┐
         │   End   │
         └─────────┘
```

## FIG. 20

```
                    ┌─────────┐
                    │  Start  │
                    └─────────┘
                         │
    ┌────────────────────┘
    │      ┌──────────────────────────────────────┐
    │      │  Receive state information associated │
    │      │  with motion of walking assistance device│──── 2010
    │      └──────────────────────────────────────┘
    │                    │
    │      ┌──────────────────────────────────────┐
    │      │         Detect preset event          │──── 2020
    │      └──────────────────────────────────────┘
    │                    │
    │      ┌──────────────────────────────────────┐
    │      │   Evaluate reward value of information │
    │      │        associated with motion         │──── 2030
    │      └──────────────────────────────────────┘
    │                    │
    │                    ▼           2040
    │              ╱─────────────╲
    │        No   ╱    Update     ╲
    └───────────◄  gait policy based on reward  ►
                 ╲     value?     ╱
                  ╲─────────────╱
                         │ Yes
      ┌──────────────────────────────────────┐
      │          Output notification         │──── 2050
      └──────────────────────────────────────┘
                         │
                    ┌─────────┐
                    │   End   │
                    └─────────┘
```

**FIG. 21**

# FIG. 22

## FIG. 23

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 18 18 2037

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/056211 A1 (LIM BOKMAN [KR] ET AL) 2 March 2017 (2017-03-02) | 1-6,8,9, 13-15 | INV. G16H20/30 |
| Y | * abstract * | 7 | A61B5/11 |
| | * paragraph [0060] - paragraph [0077] * | | A61H3/00 |
| | * paragraph [0093] - paragraph [0104] * | | A61H1/02 |
| | * claims 1-3,6-8,12-17 * | | |
| | * figures 1-3,6,7 * | | |
| | ----- | | |
| | -/-- | | |

TECHNICAL FIELDS
SEARCHED      (IPC)

A61H
G16H
A61B

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 31 May 2019 | Hilbig, Matthias |

EPO FORM 1503 03.82 (P04E07)

Europäisches
Patentamt

European
Patent Office

Office européen
des brevets

**PARTIAL EUROPEAN SEARCH REPORT**

Application Number

EP 18 18 2037

| | DOCUMENTS CONSIDERED TO BE RELEVANT | | | CLASSIFICATION OF THE APPLICATION (IPC) |
|---|---|---|---|---|
| Category | Citation of document with indication, where appropriate, of relevant passages | | Relevant to claim | |
| Y | LIM BOKMAN ET AL: "An event-driven control to achieve adaptive walking assist with gait primitives", 2015 IEEE/RSJ INTERNATIONAL CONFERENCE ON INTELLIGENT ROBOTS AND SYSTEMS (IROS), IEEE, 28 September 2015 (2015-09-28), pages 5870-5875, XP032832432, DOI: 10.1109/IROS.2015.7354211 [retrieved on 2015-12-11] * abstract * * page 5871, left-hand column, paragraph 1 - page 5873, left-hand column, paragraph 4 * * page 5874, left-hand column, last paragraph - right-hand column, last paragraph * * figures 1,2 * ----- | | 7 | |
| | | | | TECHNICAL FIELDS SEARCHED (IPC) |

EPO FORM 1503 03.82 (P04C10)

| | |
|---|---|
| Europäisches Patentamt<br>European Patent Office<br>Office européen des brevets | **INCOMPLETE SEARCH**<br>**SHEET C** |

**Application Number**

EP 18 18 2037

Claim(s) completely searchable:
       1-9, 13-15

Claim(s) not searched:
       10-12

Reason for the limitation of the search:

Claims 1, 10 and 11 have been drafted as separate independent method claims, and claims 12 and 14 have been drafted as separate independent product claims.Under Article 84 in combination with Rule 43(2) EPC, an application may contain more than one independent claim in a particular category only if the subject-matter claimed falls within one or more of the exceptional situations set out in paragraph (a), (b) or (c) of Rule 43(2) EPC. This is not the case in the present application because the subject-matter of the application does neither involve a plurality of inter-related products, nor different uses of a product or apparatus nor alternative solutions to a particular problem. Thus, the search has been restricted to the subject-matter of claims 1 to 9 and claims 13 to 15 as indicated by the Applicant in his letter of 01-04-2019 filed in reply to the invitation pursuant to Rule 62a(1) EPC.

## ANNEX TO THE EUROPEAN SEARCH REPORT
## ON EUROPEAN PATENT APPLICATION NO.

EP 18 18 2037

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2019

| Patent document cited in search report | | Publication date | Patent family member(s) | | Publication date |
|---|---|---|---|---|---|
| US 2017056211 | A1 | 02-03-2017 | KR | 20170027448 A | 10-03-2017 |
| | | | US | 2017056211 A1 | 02-03-2017 |

EPO FORM P0459

For more details about this annex : see Official Journal of the European Patent Office, No. 12/82